# DEMANDE DE BREVET EUROPEEN

(11) **EP 1 577 828 A2**
(43) Date de publication de la demande: **21.09.2005**
(21) Numéro de dépôt: 05290540.3
(22) Date de dépôt: 10.03.2005
(51) Int. Cl.: G06K 19/06

(54) **Procédé de marquage anti-contrefaçon d'un produit manufacturé avec un isotope stable non radioactif**

(30) Priorité: 12.03.2004 FR 0450512
(71) Demandeur: L'OREAL, 75008 Paris (FR)
(72) Inventeur: Saint-Leger, Didier, 92110 Clichy (FR)
(74) Mandataire: Lhoste, Catherine

(57) **Abrégé**

L'invention se rapporte à un procédé industriel de marquage anti-contrefaçon d'un produit cosmétique ou dermatologique, manufacturé consistant à introduire durant la fabrication industrielle du dit produit au moins un isotope stable non radioactif d'au moins un élément atomique, de telle sorte que la teneur dudit isotope dans ledit produit manufacturé soit supérieure à sa teneur naturelle, le dit isotope étant choisi parmi les isotopes naturels ayant une teneur isotopique naturelle inférieure à 1.2% et mieux inférieure à 1%.

## Description

L'invention se rapporte à un procédé industriel de marquage anti-contrefaçon d'un produit cosmétique ou dermatologique manufacturé, comprenant, durant la fabrication industrielle de ce produit, l'enrichissement d'un isotope stable non radioactif d'un élément atomique entrant dans la constitution de ce produit.

L'invention se rapporte encore à l'utilisation d'un isotope stable non radioactif enrichi d'un élément atomique, comme marqueur dans ou pour la fabrication d'un produit cosmétique ou dermatologique manufacturé.

L'enrichissement en l'un des isotopes d'un élément atomique signifie que la teneur de cet isotope dans le produit manufacturé soit supérieure à sa teneur naturelle.

Selon l'invention, l'isotope stable non radioactif enrichi est choisi parmi les isotopes naturels ayant une teneur isotopique naturelle inférieure à 1,2% et mieux inférieure à 1%.

La contrefaçon concerne de nombreux secteurs industriels comme par exemple la parfumerie, les produits pharmaceutiques et en particulier les produits dermatologiques, les produits cosmétiques.

Nombreux sont les produits qui sont victimes de contrefaçon, spécialement lorsqu'ils rencontrent un succès commercial. Il est souvent difficile de lutter contre ces tentatives de contrefaçon, par manque de moyens permettant de différencier avec sécurité le produit d'origine du produit contrefaisant et d'assurer une authentification du produit d'origine.

L'utilisation d'isotopes non radioactifs stables pour marquer des produits et des procédés d'authentification d'un produit par marquage isotopique sont connus dans l'art antérieur. On peut citer par exemple le document FR 2 673 291 qui décrit un procédé d'authentification de l'origine d'un produit constitué de composés organiques par marquage isotopique avec le carbone 13 (¹³C). Suivant ce document, la modification volontaire par ajout d'une ou de plusieurs molécules enrichies en ¹³C provoque une variation voulue de la composition isotopique du mélange de composés organiques constituant un produit, et notamment un produit obtenu industriellement, ce qui permet de constituer une signature discrète, infalsifiable, parfaitement inoffensive (à la différence des produits soumis à un marquage radioactif) et qui n'est détectable que par des moyens analytiques spécialisés, notamment par spectrométrie de masse isotopique.

On peut encore citer les documents US 4,862,143 et US 4,742,340 qui décrivent des méthodes permettant d'authentifier des articles comprenant un corps non métallique (documents financiers, billets de banque, passeports, ...) et un "label" fixé à ce corps, ledit label comprenant un élément atomique enrichi en une quantité déterminée de fer 57 (⁵⁷Fe), d'europium 151 (¹⁵¹Eu) ou d'étain 119 (¹¹⁹Sn).

L'osmium 197 (¹¹⁹Os) est également connu du document WO97/46981 pour protéger de la contrefaçon des billets de banque, des papiers de valeur.

Les utilisations et procédés précités sont tous relatifs à des isotopes stables non radioactifs dont l'abondance ou teneur isotopique naturelle est supérieure à 1%.

| **Elément** | **Abondance isotopique naturelle** |
|---|---|
| Carbone 13 | 1,108% |
| Fer 57 | 2,17% |
| Europium 151 | 47,77% |
| Etain 119 | 8,58% |
| Osmium 187 | 1,64% |

Si la teneur isotopique naturelle des éléments isotopiques précités n'empêche nullement leur utilisation, elle a cependant comme conséquence, pour des raisons évidentes de détection, de nécessiter un enrichissement isotopique quantitativement important, afin d'assurer une détection ultérieure imparable et sûre dudit enrichissement isotopique. Cette règle, comparable à la notion de rapport signal/bruit bien connue des chimistes analytiques, entraîne des surcoûts rédhibitoires du point de vue industriel en augmentant fortement le prix de revient industriel du produit fini. En outre, les isotopes dont l'abondance ou teneur isotopique naturelle est supérieure à 1,2% et en particulier supérieure à 1% ne peuvent pas être utilisés pour marquer des compositions destinées à être utilisés chez l'homme ou l'animal dont la posologie est rigoureuse. On comprend en effet que pour pouvoir détecter ultérieurement des compositions enrichies avec des isotopes dont l'abondance ou teneur isotopique naturelle est supérieure à 1,2%, voire à 1%, l'enrichissement isotopique est tel, que la posologie rigoureuse n'est pas toujours respectée.

Par ailleurs, l'incorporation de fortes quantités de ces isotopes peut finir par modifier les équilibres naturels des éléments considérés ce qui peut avoir des conséquences du point de vue environnemental.

Bien que les isotopes stables non radioactifs ayant une teneur isotopique naturelle inférieure à 1,2%, et en particulier inférieure à 1% soient connus depuis longtemps et que la contrefaçon soit un problème identifié depuis de nombreuses années, il n'a à la connaissance de la demanderesse encore jamais été décrit de procédé industriel anti-contrefaçon de produit cosmétique ou dermatologique manufacturé, par marquage isotopique avec des isotopes naturels ayant une teneur isotopique naturelle inférieure à 1,2%, voire à 1%.

Il subsiste donc le besoin le disposer de moyens permettant de marquer un produit cosmétique ou dermatologique manufacturé, simples à mettre en oeuvre, économiquement acceptables et compatibles avec les dispositions légales et environnementales en vigueurs.

L'invention se rapporte donc à un procédé industriel de marquage anti-contrefaçon d'un produit cosmétique ou dermatologique, manufacturé, consistant à introduire, durant sa fabrication industrielle, au moins un isotope stable non radioactif d'au moins un élément atomique entrant dans sa constitution, de telle sorte que la teneur dudit isotope dans ledit produit manufacturé soit supérieur à sa teneur naturelle, le dit isotope étant choisi parmi les isotopes naturels ayant une faible teneur isotopique naturelle, à savoir inférieure à 1,2% et mieux inférieure à 1%.

De façon plus précise, l'invention a pour objet un procédé industriel de marquage anti-contrefaçon d'un produit cosmétique ou dermatologique, manufacturé, contenant un milieu physiologiquement acceptable, consistant à introduire, durant sa fabrication industrielle, au moins un isotope stable non radioactif d'au moins un élément atomique entrant dans la constitution dudit produit, de telle sorte que la teneur dudit isotope dans ledit produit manufacturé soit supérieure à sa teneur naturelle, le dit isotope étant choisi parmi les isotopes naturels ayant une teneur isotopique naturelle inférieure à 1,2%, et mieux inférieure à 1%.

Au sens de la présente invention, on entend par "marquer" la manière de rendre identifiable, reconnaissable ou distinguable une molécule, une composition, un objet, un article ou un produit, cosmétique ou dermatologique, manufacturé, par rapport à une molécule, une composition, un objet, un article ou un produit cosmétique ou dermatologique, dont on souhaite connaître l'origine ou la provenance et/ou s'assurer de l'authenticité.

"Au moins un", au sens de l'invention, signifie un ou plusieurs (2, 3 ou plus).

Le procédé suivant l'invention permet, en privilégiant un enrichissement en isotope d'un élément présent naturellement à une abondance inférieure à 1,2%, et mieux inférieure à 1%, au cours de la fabrication d'un produit cosmétique ou dermatologique, manufacturé, d'introduire de faibles quantités dudit isotope et de le rendre détectable plus aisément ou plus rapidement dans des conditions économiquement acceptables.

Par "élément atomique" au sens de la présente invention, on entend un élément atomique compris dans la classification périodique des éléments chimiques.

Par "milieu physiologiquement acceptable" au sens de la présente invention, on entend un milieu compatible avec les matières kératiniques et/ou les muqueuses, humaines, et qui soit non toxique. Ce milieu peut être approprié à une application topique (ou directe) sur les matières kératiniques et/ou les muqueuses, humaines, ou à une application par voie orale. Au sens de la présente invention, on entend par "matières kératiniques humaines", la peau, y compris le cuir chevelu, et les fibres kératiniques telles que les cheveux, les cils, les sourcils, les poils, les ongles, et plus spécialement les cheveux, d'êtres humains. Par "muqueuse", on entend principalement les lèvres du visage.

Le procédé suivant l'invention présente donc l'avantage de pouvoir disposer de produits (molécule, composition, objet ou article cosmétique ou dermatologique susceptible d'être fabriqué) marqués selon une règle dite "à minima" afin de respecter au plus proche les équilibres naturels des éléments chimiques présents sur la planète, surtout s'il s'agit d'un produit de grande consommation, tout en assurant la fiabilité de la détection analytique ultérieure par les moyens analytiques connus.

Dans le cas des compositions destinées à être utilisés chez l'homme, (compositions cosmétiques ou dermatologiques), l'enrichissement isotopique "a minima" selon le procédé de l'invention présente en outre l'avantage de pouvoir disposer de compositions, détectables ultérieurement sans que leur posologie rigoureuse soit modifiée. Tel est le cas par exemple lorsque la posologie (doses à employer) porte sur des quantités très faibles d'un actif efficace à faible concentration (hormones, vitamine D, etc...). Ainsi, par exemple, les traitements substitutifs de complémentation hormonale chez la femme ménopausée impliquent l'utilisation de patchs transdermiques dans lesquels l'hormone (17β-Estradiol) diffuse lentement de manière intra cutanée, par des doses très faibles, de 25µg à 100 µg sur 24 heures. Il convient donc, en respectant aussi la nécessité de faible coût, d'utiliser le moins de marqueur isotopique possible. Ainsi, l'adjonction de 5ng (nanogrammes) à 25µg 17β Estradiol D₃ (16,16,17) référence Isotec 49,118-7, lors de la préparation du patch sera suffisante pour une détection ultérieure de contrefaçon sans modifier la posologie rigoureuse. Tel est également le cas par exemple pour une gélule cosmétique dans laquelle est incorporé des vitamines (A, E, F, C) ou leur ester (ester de vitamine C, F notamment), du β-carotène, du lycopène, ou du glucose, marqués par un isotope ayant une teneur naturelle inférieure à 1%. Par exemple l'isotope ¹⁷O pourra être incorporé à des concentrations très faibles, sans conséquence physiologique aucune sur l'organisme de la personne prenant régulièrement ce type de gélule (pour avoir bonne mine par exemple ou pour lutter contre la chute naturelle des cheveux), ce qui ne serait pas le cas avec l'utilisation d'éléments isotopique naturelle dont la teneur est supérieure à 1,2%, et en particulier à 1%, qui nécessiteraient l'incorporation, dans la gélule, de plus grandes quantités de constituants, bénéfiques pour les matières kératiniques ou les muqueuses humaines.

Le marquage isotopique selon l'invention se fera au cours de la fabrication du produit cosmétique ou dermatologique, par l'introduction volontaire et connue du seul fabricant, d'au moins un constituant, produit de départ ou intermédiaire dudit produit dont certains atomes ont été enrichis en isotopes naturels stables non radioactifs ayant une teneur isotopique naturelle inférieure à 1,2%, et mieux inférieure à 1%.

Ainsi, dans le produit fini, se trouvera donc, à l'insu de toute autre personne que le fabricant et/ou le propriétaire légal du produit, dans une proportion légèrement plus élevée un ou plusieurs atomes enrichis en isotopes naturels.

La mise en évidence de l'enrichissement isotopique qui est réalisé selon l'invention se fait par des moyens analytiques bien connus tels que la spectrométrie de masse, la fluorescence X, le SIMS (Secondary Ion Mass Spectroscopy) ou la RMN (Résonance Magnétique Nucléaire). Les protocoles pratiques à mettre en oeuvre sont par exemple ceux décrits dans les documents, WO00/79569, US 4,862,143 et/ou US 4,742,340. Au sujet des principes de détermination quantitative de l'augmentation de la teneur en isotope stable on pourra se reporter à l'adresse internet suivante http://www.uga.edu/~sisbl/stable.html.

Avantageusement, le ou les isotopes utilisés dans le procédé de l'invention ont des masses atomiques variant de 2 à 100. De préférence, ils sont choisis parmi le deutérium (²D ou ²H, isotope de l'hydrogène), l'hélium 3 (³He), le carbone 13 (¹³C), l'azote 15 (¹⁵N), l'oxygène 17 (¹⁷O), l'oxygène 18 (¹⁸O), le néon 21 (²¹Ne), le soufre 33 (³³S), le soufre 36 (³⁶S), l'argon 36 (³⁶Ar), l'argon 38 (³⁸Ar), le potassium 40 (⁴⁰K), le calcium 42 (⁴²Ca), le calcium 43 (⁴³Ca), le calcium 46 (⁴⁶Ca), le calcium 48 (⁴⁸Ca), le fer 58 (⁵⁸Fe), le nickel 64 (⁶⁴Ni), le zinc 70 (⁷⁰Zn), le sélénium 74 (⁷⁴Se), le krypton 78 (⁷⁸Kr), le strontium 84 (⁸⁴Sr). Très préférentiellement, l'isotope stable non radioactif est choisi parmi le deutérium, l'azote 15, l'oxygène 17, l'oxygène 18, le soufre 36, le Calcium 46, le carbone 13 (¹³C).

Avantageusement, l'enrichissement dudit isotope stable non radioactif se traduit dans ledit produit cosmétique ou dermatologique manufacturé par un rapport teneur finale de l'isotope / teneur naturelle qui varie de 1,01 à 10⁶, de préférence de 1,02 à 10000, très préférentiellement de 1,05 à 5000.

Selon l'invention, le constituant, le produit de départ ou l'intermédiaire marqué est par exemple une molécule simple ou complexe, d'origine organique ou minérale, solide, liquide ou gazeuse. Avantageusement, il s'agit :
- pour les parfums, de l'éthanol ou de produits organiques aromatiques;
- pour les produits de maquillages (vernis à ongles notamment), de pigments (oxyde de fer ou d'alumine par exemple), d'agents de coalescence (latex) ou de solvants;
- pour les produits cosmétiques en général à application topique (produits de soin de la peau et produits capillaire notamment), de corps gras, de solvants hydrosolubles, de préférence l'éthanol; de silicones; de colorants capillaires, de préférence un colorant direct, un pigment minéral, organique ou nacré; de précurseurs de colorants d'oxydations; de coupleurs ou modificateurs de coloration; de tensioactifs; de conservateurs; de polymères, de préférence un polymère filmogène; d'agents défrisant ou de permanentes; d'agents oxydants, de préférence l'eau oxygénée; d'agent réducteurs; d'actifs cosmétiques comme les filtres solaires, les agents hydratants, les agents pigmentant cutanés, les agents astringents, les agents antipelliculaires;
- pour les crèmes ou pommades dermatologiques, de principes actifs, ou d'excipients,
- pour les compléments oraux à visée cosmétique, des actifs ingérables tels que les vitamines, les huiles d'origine végétale, les caroténoïdes (lycopène ou β-carotène), des d'agents d'enrobage.

De nombreuses molécules incluant des atomes isotopiques stables ayant une teneur isotopique naturelle inférieure à 1,2%, et mieux inférieure à 1%, sont disponibles commercialement, à de grande pureté d'enrichissement. Par exemple, la Société Isotec, dont le catalogue est disponible sur Internet (http://www.sigmaaldrich.com) propose plus de 2000 molécules, organiques ou minérales au sein desquelles la pureté isotopique (richesse de la teneur en cet élément au sein de la molécule) peut atteindre 99%.

La donnée isotopique ne concerne que l'état constitutif du noyau de l'atome isotopique stable (neutrons + protons) et nullement son cortège électronique qui caractérise l'activité chimique dudit atome. Il s'en suit que les propriétés intrinsèques de la molécule sous forme enrichie seront rigoureusement comparables à celle de la molécule ne contenant que des isotopes naturels.

Au sens de la présente invention, on entend par "produit", toute molécule, composition, objet ou article cosmétique ou dermatologique susceptible d'être fabriqué.

Avantageusement, le procédé selon l'invention consistera à obtenir des produits cosmétiques ou dermatologiques marqués, destinés à entrer en contact avec les matières kératiniques humaines (peau, cheveux, poils, cils, sourcils, ongles) ou les muqueuses humaines telles que les lèvres du visage (soit par application topique, soit par ingestion). Très préférentiellement le produit est une composition cosmétique ou dermatologique à application topique (ou directe).

Dans le domaine cosmétique à application topique, la composition pour la mise en oeuvre du procédé selon l'invention peut notamment se présenter sous la forme :
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres, un vernis à ongles, un soin des ongles, un mascara, un mascara traitant, un eye-liner;
- d'une composition de protection solaire ou de bronzage artificiel;
- d'un gel ou d'une lotion après-rasage;
- d'une crème dépilatoire;
- d'une composition dermatologique;
- d'une composition solide telle qu'un savon ou un pain de nettoyage;
- d'une composition pour aérosol comprenant également un agent propulseur sous pression;
- d'une lotion de mise en plis, d'une crème ou d'un gel coiffant,
- d'une composition de teinture (notamment d'oxydation) éventuellement sous forme de shampooing colorant;
- d'une lotion restructurante pour cheveux, une composition de permanente, une lotion ou un gel antichute;
- d'une composition capillaire, notamment sous forme de crème;
- un gel protecteur solaire;
- une composition de soin du cuir chevelu, notamment anti-chute ou repousse des cheveux;
- d'une composition de lavage telle qu'un shampoing, un gel douche ou un produit de démaquillage;
- d'une composition après shampoing notamment démêlant;
- d'un produit de soin, de traitement ou de protection, des matières kératiniques, et notamment de la peau du visage ou du corps, y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil.

Pour une utilisation par voir orale, les compositions peuvent se présenter sous forme de poudre, pilule, cachet ou sirop.

Le procédé suivant l'invention peut également consister à introduire une ou plusieurs molécules qui n'entrent traditionnellement pas dans la constitution du produit final mais qui n'en modifieront pas la qualité finale intrinsèque.

La présente invention se rapporte aussi à l'utilisation d'au moins un isotope stable non radioactif d'au moins un élément atomique comme marqueur dans un produit cosmétique manufacturé ou pour la fabrication d'un produit dermatologique manufacturé, ce produit cosmétique ou dermatologique contenant un milieu physiologiquement acceptable, de telle sorte que la teneur dudit isotope dans ledit produit manufacturé soit supérieure à sa teneur naturelle, ledit isotope étant choisi parmi les isotopes naturels ayant une teneur isotopique naturelle inférieure à 1.2%.

La présente invention se rapporte encore à l'utilisation notamment cosmétique d'au moins un isotope stable non radioactif d'au moins un élément atomique comme marqueur dans un produit cosmétique manufacturé ou pour la fabrication d'un produit dermatologique manufacturé, comme agent de détection de la contrefaçon ou pour la détection de la contrefaçon dudit produit cosmétique ou dermatologique, ce produit cosmétique ou dermatologique contenant un milieu physiologiquement acceptable et ledit isotope étant présent dans ledit produit manufacturé en une teneur supérieure à sa teneur naturelle, ledit isotope étant choisi parmi les isotopes naturels ayant une teneur isotopique naturelle inférieure à 1,2%.

La présente invention se rapporte encore à l'utilisation d'au moins un isotope stable non radioactif d'au moins un élément atomique comme marqueur dans ou pour la fabrication d'une molécule, d'un objet, d'un article ou d'un produit manufacturé, cosmétique ou dermatologique, ledit isotope étant choisi parmi les isotopes naturels ayant une teneur isotopique naturelle inférieure à 1,2%, et mieux inférieure à 1%, et étant présent dans la molécule, l'objet, l'article ou le produit manufacturé, à une teneur supérieure à sa teneur naturelle.

Le procédé suivant l'invention comprend a) au moins une étape de fabrication d'un produit cosmétique ou dermatologique, manufacturé, marqué grâce à un enrichissement d'au moins un isotope stable non radioactif d'au moins un élément atomique entrant dans la fabrication dudit produit manufacturé (et en particulier dans une des molécules ou ingrédients de la composition de ce produit manufacturé, ou encore dans une des molécules ou ingrédients du conditionnement contenant cette composition), ledit isotope étant choisi parmi les isotopes naturels ayant une teneur isotopique naturelle inférieure à 1,2%, et au moins une étape de détection de la présence ou non de l'enrichissement dudit isotope stable non radioactif.

La détection en isotope enrichi peut se faire en particulier par analyse par RMN, SM ou SIMS.

Le procédé suivant l'invention peut notamment être illustré par les exemples d'application suivants:

### 1) Composition cosmétique parfumante

L'utilisation d'éthanol comme le CD₃CD₂OD (ref Isotec 18,641-4) ou le CH₃CH₂¹⁷OH (ISOTEC 58,629-3) ou encore le CH₃CH₂¹⁸OH (Isotec 60,987-0) permet de réaliser des compositions cosmétiques parfumantes marquées. Du point de vue analytique, la détermination, après séparation chromatographique de la fraction éthanolique, montrera une claire augmentation des teneurs en D, ¹⁷O ou ¹⁸O. Deux exemples plus précis de réalisation de compositions cosmétiques parfumantes utilisables selon l'invention sont donnés ci-après.

| Composition cosmétique parfumante n°1 (en %, poids/poids) | |
|---|---|
| Essence parfumante (mélange d'esters et d'adéhydes naturels) | 8,00 g |
| Filtre anti UV (vendu sous le nom de Covasorb) | 1,00 g |
| Butylhydroxytoluène | 0,05 g |
| Ethanol | 50,00 g |
| CH₃CH₂¹⁸OH (Isotec 60,987-0) | 0,001 g |
| Eau | qsp 100 g |

| Composition cosmétique parfumante n°2 (en %, poids/poids) | |
|---|---|
| Essence parfumante (mélange d'esters et d'adéhydes naturels) | 8,00 g |
| Filtre anti UV (vendu sous le nom de Covasorb) | 1,00 g |
| Butylhydroxytoluène | 0,05 g |
| Ethanol | 50,00 g |
| CH₃CH₂¹⁷OH | 0,001 % |
| Eau | qsp 100 |

### 2) Huile entrant dans la fabrication d'une composition cosmétique

Les huiles végétales sont pour l'essentiel des mélanges plus ou moins variables de Triglycérides portant des acides gras de nature et de longueur de chaînes différentes. Parmi celles-ci, la Tripalmitine (3 acides palmitique estérifiés par les 3 hydroxyles du Gycérol) est un Triglycéride le plus classiquement rencontré.
Un producteur d'huiles végétales (Palme, Olive, Tournesol, Macadamia, Monoï...) pourra par exemple introduire dans sa production, de 0,01 à 0,05% de Glyceryl Trihexadecanoate, 16,16, 16D₃ (Isotec 61,547-1), c'est-à-dire de la Tripalmitine dans laquelle tous les hydrogènes terminaux des chaînes grasses sont des Deutérium. On peut encore citer le Glyceryl Trihexadecanoate D₃₁ (Isotec 61,696-6), c'est-à-dire de la Tripalmitine dans laquelle tous les Hydrogène des chaînes grasses sont des Deutérium.
Ainsi, l'analyse d'une composition contenant une huile qui contiendrait naturellement 5% en poids de Tripalmitine verrait, dans le cas par exemple d'un ajout de 0,02% de Glyceryl Trihexadecanoate D31 en poids de l'huile entière, une augmentation nette de la teneur en Deutérium, au niveau de ce Triglycéride, passant d'une teneur en deutérium de 0.015% à 0.37% environ ; une technique comme la GC-MS ne manquerait pas de constater cet enrichissement.

### 3) Gélule à base d'amidon ou de gélatine enrichi en nutriment (glucose, vitamines, caroténoïdes, minéraux)

Dans de nombreuses compositions cosmétiques ingérables, l'actif est enrobé dans une gélule dure, dont le poids peut varier par exemple de 500mg à 1 gramme et dont l'Amidon (ou la gélatine) sont des constituants majeurs, de 95 à 99%, le reste étant des agents liants, des colorants.
L'introduction au sein de la pâte de la gélule, avant enrobage de l'actif, par exemple de glucose C₆D₁₂0₆ (Isotec 61,633-8) à un taux de 0,001% de celle-ci, permet un marquage de la composition ingérable, sans entraîner de forts coûts industriels. L'analyse de la gélule ainsi créée sera conduite par solubilisation de celle-ci, puis l'isolement du glucose ainsi libéré. Des techniques analytiques classiques RMN ou spectrométrie de masse permettront de vérifier sa composition isotopique.

### 4) Composition de maquillage à base de pigments inorganiques

Dans la préparation des composition de maquillage, des pigments tels que les oxydes d'aluminium et ferrique sont introduits le plus souvent à des concentrations de 0,5 à 12%, par exemple de 0,5 à 4%.
Le remplacement de 0,02% d'oxyde d'aluminium Al₂O₃ (sur 2% d'oxyde d'aluminium total utilisé par exemple), au cours de la préparation de la composition, par de l'oxyde d'aluminium Al₂¹⁷O₃ (Isotec 60,993-5) dans lequel les 3 atomes d'oxygène sont sous forme ¹⁷O, conduit à une teneur totale de 1,003% en ¹⁷O au lieu de 0,037% initial, aisément détectable par spectrométrie de masse. De manière tout autant comparable, on peut procéder aussi au choix de l'oxyde de fer Fe₂¹⁷O₃ ( Isotec 58,917-9), en remplacement d'une partie du même oxyde de fer comportant de l'oxygène sous forme naturelle 16, la plus abondante.
Un produit de maquillage fabriqué avec de tel pigment sera par conséquent enrichi en ces éléments et pourra ainsi être identifié/authentifié par l'utilisation de la méthode SI-MS décrite précédemment.

### 5) Compositions cosmétiques pour cheveux

### Préparation colorante pour cheveux à base de ¹⁵N₂ PPD

Synthèse préalable de ¹⁵N₂ Para Phénylène Diamine (PPD ou 1,4 diaminobenzène) par simple réduction de la ¹⁵N₂ Para Nitro Aniline dont les 2 atomes d'azote sont sous forme ¹⁵N (commercialisée par la Société Isotec/Sigma Aldrich, référence 48,773-2).

Dans un hydrogénateu,r on place 5 grammes de ¹⁵N₂ para Nitroaniline, 8 g de palladium à 5% sur charbon actif (contenant 50% d'eau), 150 ml d'éthanol à 96° et 150 ml d'eau.
La réduction est faite en une demi-heure sous une pression d'hydrogène d'environ 8 bars et à une température qui a été progressivement portée à 75°C.
Après filtration du catalyseur (Pd) sous azote on évapore le filtrat à sec sous pression réduite.
Le composé cristallisé obtenu est ensuite purifié par recristallisation dans l'éthanol au reflux. On obtient ainsi une Paraphénylène Diamine dont les deux groupements azotés (NH₂) sont tous deux constitués par des isotopes stables de l'azote 15.

| Préparation colorante | |
|---|---|
| Paraphénylène diamine | 0,1500 g |
| ¹⁵N₂ PPD (¹⁵N₂ 1,4- diaminobenzène) | 0,0015 g |
| 1-Amino 4-hydroxybenzène | 0,0500 g |
| 1,3-Dihydroxybenzène | 0,1500 g |
| 1-Hydroxy 3- aminobenzène | 0,1500 g |
| 2-N-Méthylamino 4 β-hydroxyéthyloxy niitrobenzène | 0,0500 g |
| Tertiobutylhydroquinone | 0,1660 g |
| Bisulfite de Sodium à 35° Bé | 1,5000 g |
| Alcool oléique glycérolé à 2 moles de glycérol | 5,0000 g |
| Alcool oléique glycérolé à 4 moles de glycérol | 5,0000 g |
| Acide oléique | 5,0000 g |
| Diéthanolamine oléique | 5,0000 g |
| Diéthanolamide oléique | 12,0000 g |
| Alcool éthylique | 10,0000 g |
| Ethoxy-2 éthanol | 12,0000 g |
| Acide éthylènediaminotetracétique | 0,2000 g |
| Ammoniaque à 22° Bé | 10,2000 g |
| Eau | qsp 100 g |

Cette composition est un liquide que l'on mélange au moment de l'emploi avec son poids d'eau oxygénée à 20 volumes.

| Composition lavante pour cheveux sous forme de Shampooing (en %, poids/poids) | |
|---|---|
| Laureth 12 | 0,250 |
| Sodium Laureth Sulfate | 8,400 |
| D₂₅Sodium Lauryl Sulfate (Isotec 45,185-1) | 0,001 |
| Sodium Methyl Paraben | 0,200 |
| Propylène Glycol | 0,450 |
| DMDM Hydantoïne | 0,110 |
| Cocamide MAE | 0,500 |
| Coco Bétaïne | 3,000 |
| Sodium Chloride | 1,650 |
| Parfum | 0,500 |
| Acid tallow 3 (colorant) | 0,002 |
| Eau | qsp 100 |

### Formule capillaire nutritive à base de 6-Octadécénoate de Glucose

La synthèse préliminaire du 6-Octadécénoate de Glucose peut être réalisée suivant le procédé de synthèse décrit dans le document EP1371658 à partir de l'acide ¹³C₁₈ linoléique (tous les 18 atomes de carbone étant sous forme ¹³C) et de D glucose ¹³C₆ (tous les 6 atomes de carbone étant sous forme ¹³C), les deux composés étant commercialement disponibles chez la Société Isotec/Sigma Aldrich. Les 24 atomes de carbone constitutifs de l'ester ainsi obtenu sont sous la forme ¹³C.

| | |
|---|---|
| ¹³C₂₄ -6 Octadécénoate de Glucose | 0,5 g |
| Huile de Ricin hydrogénée oxyéthylénée 40 | 0,5 g |
| Butylhydroxytoluène | 0,1 g |
| Ethanol | 50,7g |
| Parfum | qs |
| Eau | qsp100g |

### 6) Compositions cosmétiques pour la peau

| Composition de Crème hydratante visage (en %, poids/poids) | |
|---|---|
| Cetyl alcool | 7,00 |
| ¹²C Glycérol | 5,30 |
| ¹³C₃ Glycérol (Isotec 48,947-6) | 2,00 |
| Mineral oil | 2,00 |
| PEG-50 Stéarate | 2,50 |
| Acide Stéarique | 0,13 |
| Glyceryl Stearate SE | 2,25 |
| Isopropyl Palmitate | 3,00 |
| Fragrance | 0,30 |
| Methyl Paraben (conservateur) | 0,30 |
| Eau | qsp 100 |

## Revendications

1. Procédé industriel de marquage anti-contrefaçon d'un produit cosmétique ou dermatologique, manufacturé, contenant un milieu physiologiquement acceptable, consistant à introduire durant sa fabrication industrielle au moins un isotope stable non radioactif d'au moins un élément atomique entrant dans la constitution dudit produit, de telle sorte que la teneur dudit isotope dans ledit produit manufacturé soit supérieure à sa teneur naturelle, ledit isotope étant choisi parmi les isotopes naturels ayant une teneur isotopique naturelle inférieure à 1,2%.

2. Procédé suivant la revendication précédente, **caractérisé en ce que** l'isotope a une masse atomique variant de 2 à 100.

3. Procédé suivant l'une quelconque des revendications précédentes, **caractérisé en ce que** l'isotope est choisi parmi le deutérium (²D), l'hélium 3 (³He), le carbone 13 (¹³C), l'azote 15 (¹⁵N), l'oxygène 17 (¹⁷O), l'oxygène 18 (¹⁸O), le néon 21 (²¹Ne), le soufre 33 (³³S), le soufre 36 (³⁶S), l'argon 36 (³⁶Ar), l'argon 38 (³⁸Ar), le potassium 40 (⁴⁰K), le calcium 42 (⁴²Ca), le calcium 43 (⁴³Ca), le calcium 46 (⁴⁶Ca), le calcium 48 (⁴⁸Ca), le fer 58 (⁵⁸Fe), le nickel 64 (⁶⁴Ni), le zinc 70 (⁷⁰Zn), le sélénium 74 (⁷⁴Se), le krypton 78 (⁷⁸Kr), le strontium 84 (⁸⁴Sr).

4. Procédé suivant l'une quelconque des revendications précédentes, **caractérisé en ce que** l'isotope est choisi parmi le deutérium, l'azote 15, l'oxygène 17, l'oxygène 18, le soufre 36, le calcium 46, le carbone 13 (¹³C).

5. Procédé suivant l'une quelconque des revendications précédentes, **caractérisé en ce que** l'enrichissement dudit isotope stable non radioactif se traduit dans le dit produit manufacturé par un rapport teneur finale de l'isotope / teneur naturelle qui varie de 1,01 à 10⁶.

6. Procédé suivant l'une quelconque des revendications précédentes, **caractérisé en ce que** l'enrichissement dudit isotope stable non radioactif se traduit dans le dit produit manufacturé par un rapport teneur finale de l'isotope / teneur naturelle qui varie de 1,05 à 5000.

7. Procédé suivant l'une quelconque des revendications précédentes, **caractérisé en ce que** le produit est une molécule, une composition, un objet, un article.

8. Procédé suivant l'une quelconque des revendications précédentes, **caractérisé en ce que** ledit isotope est choisi parmi les isotopes naturels ayant une teneur isotopique naturelle inférieure à 1%.

9. Procédé suivant l'une quelconque des revendications 1 à 7, **caractérisé en ce que** l'isotope est le carbone 13 (¹³C).

10. Procédé suivant l'une quelconque des revendications 1 à 8, **caractérisé en ce qu'**il comprend a) au moins une étape de fabrication d'un produit cosmétique ou dermatologique, manufacturé, marqué grâce à un enrichissement d'au moins un isotope stable non radioactif d'au moins un élément atomique entrant dans la fabrication dudit produit manufacturé (et en particulier dans une des molécules ou ingrédients de la composition de ce produit manufacturé, ou encore dans une des molécules ou ingrédients du conditionnement contenant cette composition), ledit isotope étant choisi parmi les isotopes naturels ayant une teneur isotopique naturelle inférieure à 1,2%, et au moins une étape de détection de la présence ou non de l'enrichissement dudit isotope stable non radioactif.

11. Utilisation d'au moins un isotope stable non radioactif d'au moins un élément atomique comme marqueur dans un produit cosmétique manufacturé ou pour la fabrication d'un produit dermatologique manufacturé, ce produit cosmétique ou dermatologique contenant un milieu physiologiquement acceptable, de telle sorte que la teneur dudit isotope dans ledit produit manufacturé soit supérieure à sa teneur naturelle, ledit isotope étant choisi parmi les isotopes naturels ayant une teneur isotopique naturelle inférieure à 1,2%.

12. Utilisation d'au moins un isotope stable non radioactif d'au moins un élément atomique comme marqueur dans un produit cosmétique manufacturé ou pour la fabrication d'un produit dermatologique manufacturé, comme agent de détection de la contrefaçon ou pour la détection de la contrefaçon dudit produit cosmétique ou dermatologique, ce produit cosmétique ou dermatologique contenant un milieu physiologiquement acceptable, ledit isotope étant présent dans ledit produit manufacturé en une teneur supérieure à sa teneur naturelle, ledit isotope étant choisi parmi les isotopes naturels ayant une teneur isotopique naturelle inférieure à 1,2%.

13. Utilisation suivant la revendication 11 ou 12, **caractérisée en ce que** l'isotope est choisi parmi le deutérium, l'azote 15, l'oxygène 17, l'oxygène 18, le soufre 36, le calcium 46, le carbone 13 (¹³C).

14. Utilisation suivant l'une quelconque des revendications 11 à 13, **caractérisée en ce que** ledit isotope est choisi parmi les isotopes naturels ayant une teneur isotopique naturelle inférieure à 1%.

15. Utilisation suivant l'une des revendication 11 à 14, **caractérisée en ce que** l'isotope est le carbone 13 (¹³C).

16. Utilisation suivant l'une des revendications 11 à 16, **caractérisée en ce que** ledit produit cosmétique ou dermatologique, manufacturé est une molécule, un objet, un article ou un produit manufacturé, cosmétique ou dermatologique.
